# EUROPEAN PATENT SPECIFICATION

(11) **EP 0 593 809 B1**
(45) Date of publication and mention of the grant of the patent: **15.03.2000**
(21) Application number: 92118067.5
(22) Date of filing: 22.10.1992
(51) Int. Cl.: A61L 9/14, A61L 9/12

(54) **Deodorant comprising an o/w emulsion and a phase constituted by perfume microcapsules, and method of preparation**
Deodorant enthaltend eine O/W Emulsion und eine Parfummikrokapseln enthaltende Phase, und Verfahren zu ihrer Herstellung
Déodorant comprenant une émulsion H/E et une phase constituée de microcapsules de parfum, et le procédé de préparation

(43) Date of publication of application: 27.04.1994
(73) Proprietor: TAVOLA S.p.A., 20141 Milano (IT)
(72) Inventor: Moreno, Vittorio, Arese (MI) (IT)
(74) Representative: La Ciura, Salvatore

(56) References cited:
- EP-A- 0 317 658
- WO-A-90/10436
- WO-A-92/00722
- DATABASE WPIL Week 9103, Derwent Publications Ltd., London, GB; AN 91-019501& JP-A-2 293 041 (MATSUMOTO YUSHI SEIYAKU)
- DATABASE WPIL Week 9234, Derwent Publications Ltd., London, GB; AN 92-281906& JP-A-4 194 163 (TOYO LINOLEUM KK)

## Description

The invention concerns a deodorant, consisting of a water emulsion of a perfume oil, and of a certain quantity of microcapsules, filled with the same perfume oil, and mixed with the water emulsion of the perfume.

This way, an immediate release of perfume is obtained when spraying the emulsion in the environment, followed by a prolonged release of perfume, when the microcapsules deposited on floors or seats by gravity, are broken through a mechanical action, such as being walked on or when one sits on the seat itself.

The invention also concerns the method of preparation of the deodorant.

Several types of room deodorants are known and available in various forms. For example, there are deodorants in which a cellulose support is impregnated with perfume which slowly evaporates in the air.

There are also deodorants in which the perfume is mixed with other components in order to obtain a gel as well as deodorants made by plastic materials of various types.

Then, there exist liquid deodorants which can be sprayed directly in the air or deodorants which release perfume through a porous surface, or else deodorants in granular form.

The above types of deodorants, with the exception of the liquids sprayed in the air, are capable of a slow, continuous release of perfume, once they are put in use for a certain period of time.

Although these deodorants were developed to provide a certain amount of perfume in the air, they are not able to release a sufficient amount of perfume to deodorize the air in a short time, when, for example, it is desirable to eliminate objectionable odours such as cigarette smoke, cooking odours, etc.

In such case "instantaneous" deodorants are normally used, consisting of a liquid formula which is finely sprayed in the room to be deodorized.

These deodorants are sprayed either by means of a manual pump or, in the well-known aerosol form, by a compressed or liquified propellant gas.

These liquid deodorants are generally hydroalcoholic formulae, so that the sprayed product is effective for a limited length of time, depending on the volatility of the components of the formula and of the perfume itself. This time is always rather short, especially taking into account the fact that the products rapidly tend to deposit on the ground by gravity.

WO-A-92 00 722 relates to a fragrance composition comprising a fragrant base in the microencapsulated form. This composition is specifically provided for topic use.

The present invention proposes a deodorant composed of a liquid phase constitued by a water emulsion of a perfume oil and a phase constitued by microcapsules containing the same perfume oil.

The invention also concerns the relevant method of preparation.

According to the invention, the deodorant provides the advantage of a longer-lasting effect, as compared to the normal deodorants, as, beside the immediate release of perfume at the moment of its dispersion in the environment, a further effect is provided, owing to the release of perfume from the microcapsules, when, once deposited on various surfaces, they get broken when walking on them, or by other means.

The scope of the invention is that of providing a deodorant which overcomes the limitations of the usual deodorants by immediately perfuming the air when sprayed, and then by mantaining for a relatively long time its capacity to release perfume.

To this end, the invention provides a deodorant composed of a liquid phase, constitued by a water emulsion of perfume oil, and of a certain amount of microcapsules of the same perfume oil which are sprayed in the air mixed with the rest of the product, and which then tend to fall down by gravity.

The water emulsion phase of the deodorant serves the purpose of immediately perfuming the air, while the microcapsules provide a longer-lasting action as they release their perfume when they are mechanically broken as they are walked on or by other means.

The invention will be better understood by reading the following example:

### FORMULATION

The deodorant is constituted by a water emulsion of a perfume oil, to which a certain quantity of microencapsulated perfume has been added and held in suspension within the perfume emulsion in water.

A formula suitable for the purpose of the invention is as follows:

| | Preferred Composition |
|---|---|
| Perfume 0.5% - 10% | 3% |
| Emulsifier 0.2% - 10% | 2% |
| Microencapsulated Perfume 0.2% - 10% | 2% |
| Preservative | As needed |
| Demineralized | Water Balance to 100% |

This perfume will have to be chosen among those which are non-miscible with water.

Therefore, those perfumes which more or less spontaneously become emulsified in water must be excluded. Such characteristic is mandatory because only non-emulsifible perfumes are suitable to be microencapsulated according to the usual techniques.

The normally used emulsifiers which are useful to disperse perfumes in water can be used for the purpose of the present invention.

Based on the tests which were performed, the best results were obtained with BASF'S Cremophor RH 40® Emulsifier.

For every selected perfume, those expert in the art can easily find the quantity of emulsifier which is needed to provide a stable emulsion.

This emulsion is usually a milky-white liquid, or in rarer rases, an opalescent liquid.

It is always preferable to use the lowest level of emulsifier capable of providing a stable emulsion for a period of at least six months at room temperature.

The preservative used must guarantee an effective protection of the product against a wide range of bacterial strains and yeasts.

Among the different preservatives which were tested, it was shown that the most effective is Kathon CG®, produced by Sandoz, used at very low levels, between 3ppm and 10ppm, preferably 5ppm.

The microcapsules containing the perfume are constitued by the perfume itself, finely dispersed in microscopic droplets coated by gelatine.

The microencapsulation process is not described as it can be carried out through known techniques.

The microcapsules of perfume and gelatine contain between 50% and 90% perfume, preferably between 80% and 85% perfume, and have sizes ranging from 1 to 10 micron, preferably 5 micron.

The microcapsules are used under the form of a water suspension ("slurry"), containing between 10% and 30% microcapsules, preferably 20%.

Thus, if it is desired to obtain a finished product containing 2% perfume in the microcapsules, and microcapsules at 83% perfume in a 20% "slurry" are available, 12.048% of such "slurry" should be used in the making of the finished product.

As the microcapsules in suspension in water tend to sediment it is necessary to keep them under moderate agitation during the preparation of the finished product.

The finished product is prepared as follows:
- Premix the perfume and the emulsifier.
- Add the water under moderate agitation.
- Add the microcapsule "slurry" under moderate agitation.
- Add the preservative.

The deodorant product according to the present invention provides, as stated above, the dual advantage of an immediate deodorant/perfuming action and of a further continuous release of perfume, thanks to mechanical break-up of the microcapsules when they were walked on, etc.

Such characteristics make this deodorant product particulary helpful for use in automobiles, homes, offices, public places, etc.

A further advantage is due to the fact that the product does not contain solvents, thus overcoming possible risks of flammability and other possible drawbacks related to the use of solvents.

## Claims

1. A room deodorant characterized by the fact that it is constitued by a liquid phase, comprising an emulsion in water of a perfume oil and another phase constitued by microcapsules containing the same perfume oil, which are mixed with the above water emulsion.

2. Deodorant according to claim 1, in which the perfume is present in quantities ranging between 0.5% and 10%, preferably 3%, the emulsifier is present between 0.2% and 10%, preferably 2%, and the perfume in the microcapsules between 0.2% and 10%, preferably 2%.

3. Process for the production of a deodorant according to claims 1 and 2, comprising the following steps:
- Mixing perfume and emulsifier.
- Adding water under moderate agitation.
- Preparing perfume containing microcapsules.
- Adding water suspension of perfume microcapsules to the water emulsion of perfume under moderate agitation.
- Adding a preservative.

## Patentansprüche

1. Raumdeodorant, dadurch gekennzeichnet, daß es aus einer flüssigen Phase aufgebaut ist, umfassend, eine Emulsion eines Parfümöls in Wasser und eine weitere aus das gleiche Parfümöl enthaltenden Mikrokapseln, welche mit der vorstehend genannten Wasseremulsion vermischt sind, aufgebaute Phase.

2. Deodorant nach Anspruch 1, wobei das Parfüm in Mengen im Bereich zwischen 0,5% und 10%, vorzugsweise 3% vorliegt, der Emulgator zwischen 0,2% und 10%, vorzugsweise 2% vorliegt und das Parfüm in den Mikrokapseln zwischen 0,2% und 10%, vorzugsweise 2% vorliegt.

3. Verfahren zur Herstellung eines Deodorants nach Ansprüchen 1 und 2, umfassend die nachstehenden Schritte:
- Vermischen von Parfüm und Emulgator,
- Zugeben von Wasser unter mäßiger Bewegung,
- Herstellen von Parfüm-enthaltenden Mikrokapseln,
- Zugeben der Wassersuspension von Parfümmikrokapseln zu der Wasseremulsion des Parfüms unter mäßiger Bewegung,
- Zugeben eines Konservierungsmittels.

## Revendications

1. Désodorisant ambiant caractérisé en ce qu'il est constitué d'une phase liquide, comprenant une émulsion dans l'eau d'une huile de parfum et d'une autre phase constituée de microcapsules contenant la même huile de parfum, qui sont mélangées à l'émulsion aqueuse ci-dessus.

2. Désodorisant suivant la revendication 1, dans lequel le parfum est présent en des quantités allant entre 0,5 % et 10 %, avantageusement 3 %, l'émulsifiant est présent entre 0,2 % et 10 %, avantageusement 2 %, et le parfum dans les microcapsules entre 0,2 % et 10 %, avantageusement 2 %.

3. Procédé de production d'un désodorisant suivant l'une ou l'autre des revendications 1 et 2, comprenant les étapes suivantes :
- mélanger du parfum et de l'émulsifiant;
- ajouter de l'eau sous une agitation modérée;
- préparer des microcapsules contenant du parfum;
- ajouter une suspension aqueuse de microcapsules de parfum à l'émulsion aqueuse de parfum sous une agitation modérée;
- ajouter un agent de conservation.
